# EUROPÄISCHE PATENTANMELDUNG

(11) **EP 1 324 041 A1**
(43) Veröffentlichungstag der Anmeldung: **02.07.2003**
(21) Anmeldenummer: 02024340.8
(22) Anmeldetag: 02.11.2002
(51) Int. Cl.: G01N 33/543

(54) **Testsystem zur Untersuchung einer biologischen Flüssigkeit**

(30) Priorität: 06.11.2001 DE 10153963
(71) Anmelder: Aventis Behring GmbH, 35002 Marburg (DE)
(72) Erfinder: Lengsfeld, Thomas, Dr., 35041 Marburg (DE)

(57) **Zusammenfassung**

Beschrieben wird ein Testsystem zur Untersuchung einer biologischen Flüssigkeit, welches aus einem schlauch- oder röhrenförmigen Hohlkörper besteht, der zur Aufnahme oder Durchleitung einer biologischen Flüssigkeit bestimmt ist und auf dessen Innenseite ein Träger befestigt ist, auf dem in voneinander abgetrennten Feldern Bindemoleküle für ein oder mehrere unterschiedliche, in der biologischen Flüssigkeit nachzuweisende Antigene, Antikörper, Pathogene, Toxine und/oder körperfremde oder körpereigene Proteine angeordnet sind.

## Beschreibung

Gegenstand der Erfindung ist ein Testsystem zum Nachweis geringer Mengen von Antigenen, Antikörpern, Pathogenen, Toxinen und/oder körperfremden oder körpereigenen Proteinen in großen Volumina biologischer Flüssigkeiten.

Es ist bekannt, dass Körperflüssigkeiten, insbesondere Blut, Plasma und Serum, in der Medizin für diagnostische und therapeutische Anwendungen gesammelt werden. Die Verwendung dieser Körperflüssigkeiten oder der daraus gewonnen Produkte für therapeutische Anwendungen ist nur möglich, wenn die Körperflüssigkeiten frei von schädigenden Substanzen wie Antigenen, Antikörpern, Pathogenen, Toxinen oder körperfremden Proteinen sind. Deshalb ist es erforderlich, die Körperflüssigkeit daraufhin zu untersuchen, ob derartige schädliche Fremdstoffe oder bestimmte Marker für derartige schädliche Fremdstoffe (Analyten) vorliegen. Diese Analyten, die häufig nur in sehr geringen Mengen vorhanden sind, können insbesondere Nukleinsäuren, Antikörper oder Antigene, aber auch Viren oder Prionen sein. Unphysiologische Konzentrationen von körpereigenen Proteinen können darüber hinaus Hinweise für das Vorhandensein von Infektionserregern oder Krankheiten sein. Zu ihrem Nachweis werden bisher kleine Fraktionen von Plasma aufgearbeitet und mittels an sich dem Fachmann bekannter Verfahren, wie z. B. Antigen-Antikörperreaktionen oder mittels der PCR auf entsprechende Pathogene getestet. Hierfür werden kleinvolumige Testmengen nach der Plasmagewinnung abgetrennt und untersucht. Die zur Verfügung stehenden Nachweismethoden sind dabei durch das Materialvolumen und die Sensitivität der eingesetzten Untersuchungsverfahren limitiert.

Gegenstand der Erfindung ist nun ein Testverfahren zum Nachweis und der Quantifizierung geringer Analytmengen in großen Flüssigkeitsvolumina, bei dem vorzugsweise die gesamte Analytmenge oder ein wesentlicher Teil mit trägergebundenen spezifischen Bindungsmolekülen binden, von der Flüssigkeit getrennt werden und anschließend in gebundenem Zustand oder auch nach Ablösung von dem Träger, mit dem Fachmann an sich bekannten Verfahren nachgewiesen oder quantifiziert werden. Üblicherweise wird dabei die Flüssigkeit relativ zu den trägergebundenen spezifischen Bindungsmolekülen bewegt, z. B. durch gravimetrischen Fluss oder Pumpen.

Eine Ausführungsform der Erfindung ist ein Testsystem zur Untersuchung einer biologischen Flüssigkeit, das aus einem schlauch- oder röhrenförmigen Hohlkörper besteht, der zur Aufnahme oder Durchleitung der biologischen Flüssigkeit bestimmt ist und auf dessen Innenseite ein Träger befestigt ist, auf dem in voneinander abgetrennten Feldern Bindemoleküle für ein oder mehrere unterschiedliche, in der biologischen Flüssigkeit nachzuweisende Antigene, Antikörper, Pathogene, Toxine und/oder körperfremde oder körpereigene Proteine angeordnet sind. Der Träger kann flächig oder gebogen sein und ist dabei in dem Testsystem so angeordnet, dass er zur Durchführung weiterer Nachweisreaktionen mit den an den Bindemolekülen fixierten Substanzen dem Hohlkörper entnommen werden kann.

Charakteristisch für das erfindungsgemäße Testsystem ist, dass die zu untersuchende biologische Flüssigkeit den schlauch- oder röhrenförmigen Hohlkörper durchfließen und dabei in intensiven Kontakt mit den auf dem Träger fixierten Bindemolekülen treten kann.

Als Bindemoleküle kommen alle chemischen organischen und anorganischen Substanzen in Frage, die die Fähigkeit haben, spezifisch ein bestimmtes Antigen, einen Antikörper, ein Pathogen, ein Toxin oder ein körperfremdes oder körpereigenes Protein zu binden. Polyklonale, monoklonale oder rekombinante Antikörper oder Antikörperfragmente eignen sich für diesen Zweck ganz besonders.

Das Material des flächigen Trägers kann aus Metall, Glas, Keramik oder einem flexiblen Werkstoff bestehen, wobei sich vor allem Cellulose-, Polyamid- und Polyestermembranen besonders bewährt haben. Zusätzlich kann der Träger mit Substanzen beschichtet sein, die die Eigenschaften der Bindemoleküle fördern. Ein derartiges Verfahren ist beschrieben von R. Jenison et al. in Clinical Chemistry 47:10,1894-1900(2001).

Der schlauch- oder röhrenförmige Hohlkörper ist im allgemeinen ein Glas- oder Plastikrohr, ein Gummi- oder Plastikschlauch oder ein Zylinder einer Injektionsspritze.

Gegenstand der Erfindung ist auch ein Verfahren zum Nachweis und/oder zur Entfernung von Antigenen, Antikörpern, Pathogenen, Toxinen und/oder körperfremden oder körpereigenen Proteinen in einer biologischen Flüssigkeit, bei dem man die Flüssigkeit mit dem schlauch- oder röhrenförmigen Hohlkörper in Kontakt bringt oder ihn durchfließen lässt, wobei die auf dem flächigen oder gebogenen Träger befestigten Bindemoleküle die nachzuweisenden Substanzen fixieren, welche dann entweder auf dem Träger im Hohlkörper oder vorzugsweise nach Entnahme des Trägers außerhalb des Hohlkörpers mit einem spezifischen Reagenz detektiert werden können.

Die an den Bindemolekülen des Trägers fixierten, zu detektierenden Substanzen können auf dem Träger mit einer physikalischen Methode, z.B. der SPR-Technik (surface plasmon resonance) der Fa. Biacore, oder vorzugsweise mittels eines weiteren, enzymatischen, fluoreszierenden oder radioaktiv markierten Bindemoleküls nachgewiesen werden.

Ein besonderer Vorteil des erfindungsgemäßen Testsystems besteht darin, dass während der Entnahme der Körperflüssigkeit aus dem Spenderorganismus kontinuierlich über einen kurzen oder langen Zeitraum hinweg die an dem mit den Bindemolekülen ausgerüsteten Träger vorbei-fließenden Markermoleküle gesammelt werden können. Da große Volumina der Körperflüssigkeit an den Bindemolekülen des Trägers vorbeifließen, können mit diesem Verfahren auch geringe Mengen an Markern, die in einem großen Flüssigkeitsvolumen sonst nur schwer oder gar nicht nachzuweisen sind, erfindungsgemäß sicher detektiert werden. Beispielsweise können Bindemoleküle für Viren, Prionen oder andere Krankheitserreger, aber auch ein Antigen wie ein Tumormarker oder ein anderer Pathomarker mittels eines Trägers in den für die Plasmagewinnung benötigten Röhren- oder Schlauchsystemen eingebaut sein. Ausgewählt werden dabei solche Bindemoleküle, die zum Beispiel Prionen oder Prionensubtypen spezifisch aus der vorbeigeleiteten Körperflüssigkeit herausfangen können. Zur Durchleitung der Körperflüssigkeit eignen sich alle Systeme, durch die große Flüssigkeitsmengen fließen können. Als biologische Flüssigkeiten kommen dabei Blut, Plasma, Serum, Milch, Lungenlavage oder Urin eingesetzt werden.

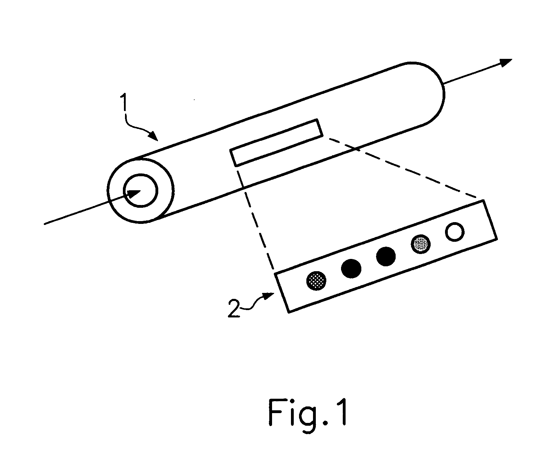

Fig. 1 zeigt ein derartiges Durchflusssystem, in dem ein flächiger Träger befestigt werden kann, auf dem in voneinander abgetrennten Feldern Bindemoleküle für verschiedene, in der Körperflüssigkeit nachzuweisende Antigene, Antikörper, Pathogene, Toxine oder körperfremde oder körpereigene Proteine angeordnet sind. Der in dem röhrenförmigen Hohlkörper (1) befestigte flächige Träger (2) kann dem Hohlkörper entnommen und für weitere Untersuchungen eingesetzt werden. Eine solche Untersuchungseinheit kann zum Beispiel auf der Protein Chip Technologie basieren. Beispielsweise kann auf dem Träger ein für Prionen spezifischer Antikörper gebunden sein. Das an dem Antikörper gebundene Prion wird dann über Sekundärreaktionen nachgewiesen. Es ist auch möglich, einzelne Nachweisfelder zunächst abzudecken und erst in späteren Tests für Kontrollverfahren einzusetzen. Außerdem können mit einzelnen Feldern zusätzlich Plasma-Referenzproteine nachgewiesen werden und als Negativkontrolle oder als ein allgemeiner Untersuchungsnachweis dienen.

Durch folgende Beispiele wird die Erfindung veranschaulicht.

### Beispiel 1

Das einem Spender entnommene Blut wird durch ein Schlauchsystem geleitet, das ein streifenförmiges Trägermaterial in der Größe von 10 x 4 mm enthält, auf dem zum Nachweis des Hepatitis C-Virus Bindemoleküle bestehend aus HCV-Antigenen C22-3, C200 und NS5 fixiert ist. Die in dem vorbeiströmenden Blut enthaltenen humanen Antikörper werden durch die Bindemoleküle gebunden und dann nachgewiesen.

### Beispiel 2

Das einem Spender entnommene Blut soll auf HIV untersucht werden. Zu diesem Zweck wird der Träger mit einem monoklonalen Antikörper gegen HIV ausgerüstet und in einem vom Spenderblut durchflossenen Schlauch befestigt. Die an dem Träger gebundenen Viruspartikel werden direkt über einen Sekundärantikörper oder über eine virusspezifische PCR-Reaktion nachgewiesen.

### Beispiel 3

Auf voneinander abgetrennten Feldern einer Nylonmembran werden Antikörper gegen verschiedene Cytokine (BD PharMingen; (San Diego, Californien)) aus einer Lösung von 100 µg/ml in Mengen von je 0,25 µl aufgetragen. An dem so vorbereiteten Träger wird 1 I Blutplasma vorbeigeleitet, dem Cytokine in einer Menge von 100 µg/ml zugefügt werden. Nach Entnahme des Trägers aus dem Hohlkörper und sorgfältigem Abwaschen von anhaftendem Serum werden die an den Bindestellen haftenden Cytokine mittels biotinylierter Cytokin-Antikörper nachgewiesen.

## Patentansprüche

1. Testsystem zur Untersuchung einer biologischen Flüssigkeit, **dadurch gekennzeichnet, dass** es aus einem schlauch- oder röhrenförmigen Hohlkörpers besteht, der zur Aufnahme oder Durchleitung der biologischen Flüssigkeit bestimmt ist und auf dessen Innenseite ein Träger befestigt ist, auf dem in voneinander abgetrennten Feldern Bindemoleküle für ein oder mehrere unterschiedliche, in der biologischen Flüssigkeit nachzuweisende Antigene, Antikörper, Pathogene, Toxine und/oder körperfremde oder körpereigene Proteine angeordnet sind.

2. Testsystem nach Anspruch 1, **dadurch gekennzeichnet, dass** die zu untersuchende biologische Flüssigkeit den schlauch- oder röhrenförmigen Hohlkörper durchfließen und dabei in Kontakt mit den auf dem Träger fixierten Bindemolekülen treten kann.

3. Testsystem nach den Ansprüchen 1 und 2, **dadurch gekennzeichnet, dass** es als Bindemoleküle polyklonale, monoklonale oder rekombinante Antikörper oder Antikörperfragmente enthält.

4. Testsystem nach den Ansprüchen 1 bis 3, **dadurch gekennzeichnet, dass** der schlauch- oder röhrenförmige Hohlkörper ein Glas- oder Plastikrohr, ein Gummi- oder Plastikschlauch oder ein Zylinder einer Injektionsspritze ist.

5. Verfahren zum Nachweis und/oder zur Entfernung von Antigenen, Antikörper, Pathogenen, Toxinen und/oder körperfremden oder körpereigenen Proteinen aus einer biologischen Flüssigkeit, **dadurch gekennzeichnet, dass** man die Flüssigkeit mit dem schlauch- oder röhrenförmigen Hohlkörper in Kontakt bringt oder ihn durchfließen lässt und dann die an den Bindemolekülen des flächigen oder gebogenen Trägers haftenden, nachzuweisenden Substanzen mit einem spezifischen Reagenz detektiert.

6. Verfahren nach Anspruch 5, **dadurch gekennzeichnet, dass** der mit den Bindemolekülen ausgerüstete Träger nach Fixierung der zu detektierenden Substanzen dem Hohlkörper entnommen und diese mit einer physikalischen Methode oder mittels eines weiteren enzymatischen, fluoreszierenden oder radioaktiv markierten Bindemoleküls nachgewiesen werden.

7. Verfahren nach den Ansprüchen 5 und 6, **dadurch gekennzeichnet, dass** als biologische Flüssigkeit Blut, Plasma, Serum, Milch, Lungenlavage oder Urin eingesetzt wird.

8. Verfahren nach den Ansprüchen 5 bis 7, **dadurch gekennzeichnet, dass** das nachzuweisende Pathogen ein Virus, ein Prion oder ein anderer Krankheitserreger ist.

9. Verfahren nach den Ansprüchen 5 bis 7, **dadurch gekennzeichnet, dass** das nachzuweisende Antigen ein Tumormarker oder ein anderer Pathomarker ist.
